# EUROPÄISCHE PATENTSCHRIFT

(11) **EP 0 587 624 B1**
(45) Veröffentlichungstag und Bekanntmachung des Hinweises auf die Patenterteilung: **13.12.1995**
(21) Anmeldenummer: 92910909.8
(22) Anmeldetag: 03.06.1992
(51) Int. Cl.: A61M 5/142

(54) **VORRICHTUNG ZUM SICHEREN BEFÜLLEN DER BEHÄLTER EINER INFUSIONSPUMPE**
DEVICE FOR RELIABLY FILLING THE CONTAINERS OF AN INFUSION PUMP
DISPOSITIF DE REMPLISSAGE FIABLE DES RECIPIENTS D'UNE POMPE A PERFUSION

(30) Priorität: 07.06.1991 DE 9107030 U
(43) Veröffentlichungstag der Anmeldung: 23.03.1994
(73) Patentinhaber: OPTON Feintechnik Kiel GmbH, 24106 Kiel (DE)
(72) Erfinder: JÜRGEN, Hinrichs, D-2300 Kiel 1 (DE); KARL-HEINZ, Otto, D-2300 Kiel 14 (DE)
(74) Vertreter: Tönnies, Jan G., Dipl.-Ing.
(86) Internationale Anmeldenummer: DE9200466
(87) Internationale Veröffentlichungsnummer: WO9221390

(56) Entgegenhaltungen:
- WO-A-89/10149
- WO-A-89/10157
- US-A- 4 861 341
- US-A- 4 892 518

## Beschreibung

Die Erfindung betrifft eine Vorrichtung zum sicheren Befüllen wenigstens zweier Behälter unter der Hautoberfläche, insbesondere einer Infusionspumpe nach dem Oberbegriff des Hauptanspruches.

Implantierbare Infusionspumpen werden zur Dauergabe von Medikamenten, z. B. Morphinen in einer gleichbleibenden Dosis über lange Zeiträume verwendet. Sie haben gegenüber gewöhnlichen Injektionen den Vorteil, daß man eine Dosis nicht mehr soweit überdosieren muß, daß trotz Abbaus des Medikamentes bis zum nächsten Verabrechungszeitpunkt eine gewisse Mindestdosis nicht unterschritten wird, sondern man eine gleichmäßige und insgesamt wesentlich verringerte Zufuhr des Medikamentes verwirklichen kann.

Dabei wird zunehmend häufiger die Möglichkeit geschaffen, mit einer Spritze in einen Behälter der Infusionspumpe eine sogenannte Bolusgabe zu verabreichen, die dann über einen bereits gelegten Katheter der Infusionspumpe an den gewünschten Ort gelangt. Sinn dieser Bolusgabe ist es, unter bestimmten medizinische Vorraussetzungen eine erhöhte Medikamentengabe für kurze Zeit zu verabeichen, wobei später die Infusionspumpe wieder ihre normale Tätigkeit fortsetzen soll.

Bisher ergeben sich jedoch beim Befüllen der Behälter im Körper Probleme. Es kann zur Zeit noch nicht mit letzter Sicherheit verhindert werden, daß eine eine neue Füllung in die Infusionspumpe spritzende Person nicht den falschen Behälter, - den für die Bolusgabe -, trifft, so daß der die Infusionspumpe tragende Patient seine (z.B. Monats-)Dosis als Bolusgabe erhält, was im Extremfall lebenbedrohend sein könnte.

Eine implantierbare Infusionsvorrichtung ist bereits aus der WO-A-89/10149 bekannt, in der die Merkmale des Oberbegriffs verwirklicht sind, bei der jedoch bei Verwendung einer falschen Nadel oder Bewegung der Nadel beim Injizieren in den falschen Behälter eingefüllt werden kann und bei Versagen einer einzigen Dichtung das Medikament, statt langsam wie vorgesehen, als Bolusgabe abgegeben wird.

Der Erfindung lag daher die Aufgabe zugrunde, eine Vorrichtung zu schaffen, die ein sicheres Nachfüllen und Geben der Bolusgabe sicherstellt und die die Septa möglichst lange funktionsfähig erhält.

Erfindungsgemäß wird dies durch die Merkmale des Hauptanspruches gelöst. Die Unteransprüche geben vorteilhafte Ausführungsformen wieder.

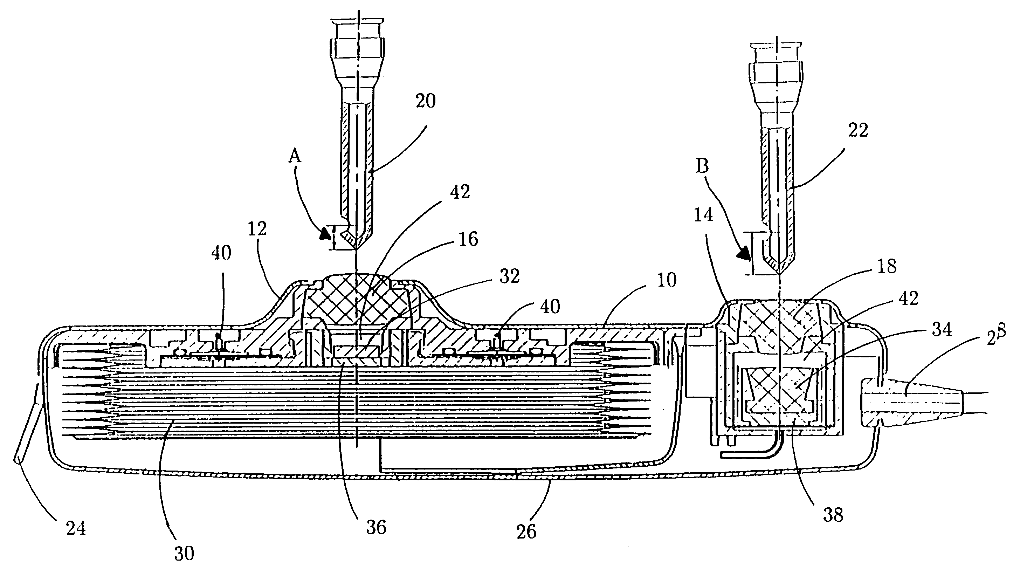

Weitere Vorteile und Merkmale der Erfindung sind in den Unteransprüchen, in der nachfolgenden Beschreibung, sowie in der Zeichnung enthalten, die ein bevorzugtes Ausführungsbeispiel der Erfindung näher erläutern. Dabei zeigt die einzige Figur eine Infusionspumpe in Schnittdarstellung mit zwei unterschiedlichen Nadeln zum Nachfüllen des Medikamentenbehälters und zur Bolusgabe über den jeweiligen Septa.

In der Figur ist in schnittdarstellung das Gehaüse 10 mit den beiden abgeflacht und abgerundet nach oben aus ihm herausragenden Ansätzen 12, 14 zur Aufnahme der Septa 16, 18 und die zu benutzenden Nadeln 20, 22 zu erkennen. Deutlich sind die beiden unterschiedlichen Distanzen A und B für die seitlichen Öffnungen der Nadeln 20, 22 von der Spitze der Nadeln dargestellt.

Die Befestigungsösen 24 an dem Gehäuseboden 26 sind nur von der Seite dargestellt. Sie sind so angeordnet, daß man mit einer Minimallänge von Faden nähen kann, so daß die Infusionspumpe nicht verrutschen kann. Von der Infusionspumpe geht dann ein Katheter 28 an den Ort, wo die Medikamentengabe geschehen soll, z. B. an die Wirbelsäule.

Je nach gewählter Fördermenge und Größe der Infusionspumpe wird sie nur in langen Zeiträumen, typisch ist ein Monat, zu befüllen sein. Dazu werden die speziellen Nadeln 20, 22 verwandt, die spanlos durch die im Deckel 10 vorgesehenen Septa 16, 18 aus Silikonkautschuk hindurchtreten können. Diese Nadeln 20, 22 haben ihre Öffnung nicht an der Spitze, sondern an der Seite in verschiedenen Abständen von der Spitze.

Die Septa 16, 18 ruhen innerhalb der Ansätze 12, 14, so daß man sie von außerhalb der Haut fühlen kann. Eine Verwechslung, etwa weil die Infusionspumpe nicht eindeutig liegt, führt mit den Nadeln 20, 22 nun nicht mehr zur falschen Medikation. Wird das falsche Septum gewählt, sperren die Dichtungen neben dem Septum die Öffnungen in der Nadel und keine Flüssigkeit kann in die Infusionspumpe gelangen.

Ein Septum 16 zum Befüllen eines Medikamentenbehälters aus einem Federbalg 30 und ein weiteres Septum 18, um eine Bolusgabe, d.h. eine sofortige Zuführung, vorzunehmen, sind nebeneinander auf dem Gehäusedeckel vorgesehen, wobei das Septum für die Bolusgabe 18 kleiner ist und weiter am Rand sitzt. Durch die nichtsymmetrische Form wird eine Unterscheidung durch Tasten von außen zur Unterstützung der spritzenden Person möglich.

Aus Festigkeitsgründen, aber auch weil das Material chemisch sehr resistent und dabei biokompatibel ist, und zusätzlich noch leicht ist, wird für alle Teile, bei denen es möglich ist, Titan verwandt.

Jeweils hinter jedem Septum ist ein Nadelstopp 36, 38 aus Kunststoff am unteren Ende jeder Septumhülse angebracht, der noch von einer Dichtung 32, 34 bedeckt wird, so daß auch keine "normalen" Nadeln mit Öffnungen in der Spitze aus Versehen den falschen Behälter füllen können.

Der Katheter 28 ist zur Seite verdrehbar an dem Gehäuse der Infusionspumpe befestigt, damit sie beim Implantieren nicht auf eine bevorzugte Körperseite festgelegt ist. Neben den Septa 16 sind zwei Betätigungselemente 40 an der Oberseite der Infusionspumpe dargestellt, mit denen die Förderrate der Infusionspumpe eingestellt werden kann.

## Patentansprüche

1. Vorrichtung zum sicheren Befüllen wenigstens zweier Behälter unter der Hautoberfläche, insbesondere einer Infusionspumpe, mit
- zwei Septa (16, 18) aus einem weichen Material, so daß zwei Nadeln (20, 22) mit seitlichen Öffnungen zum Befüllen leicht eingestochen werden können,
- zwei Nadeln (20, 22), von denen wenigstens eine eine Öffnung in einer Distanz (A; B) von der Spitze der Nadel (20; 22) besitzt,
- wenigstens jeweils einem Raum (42) zwischen weiteren Dichtungen (32, 34) und den Septa (16, 18), der mit den zu befüllenden Behältern oder Kathetern in Verbindung steht,
wobei diese Räume (42) verschieden weit von einem Nadelstopp (36, 38) entfernt sind,
dadurch gekennzeichnet, daß
- die Septa (16, 18) nebeneinander angeordnet sind,
- die zwei Nadeln jeweils eine seitliche Öffnung mit unterschiedlicher Distanz (A, B) von den Spitzen besitzen, und
- wenigstens eine der Dichtungen (16; 18; 32; 34) so ausgebildet ist, daß sie die Öffnung in einer Nadel sperrt, falls für das Septum die falsche Nadel (22; 20) gewählt ist.

2. Vorrichtung nach Anspruch 1, dadurch gekennzeichnet, daß die Septa (16, 18) aus Silikonkautschuk gefertigt sind.

3. Vorrichtung nach Anspruch 1 oder 2, dadurch gekennzeichnet, daß wenigstens eines der Septa (16, 18) an seinem nach innen weisenden Ende mit verkleinertem Durchmesser ausgebildet ist, und auf einem relativ dicken Steg ruht.

4. Vorrichtung nach einem der vorangehenden Ansprüche, dadurch gekennzeichnet, daß wenigstens eines die Septa (16, 18) an seinem nach außen weisenden Ende mit einer Kehlung versehen ist, die den Gehäusedeckel (10) oder die Wandung einer Septumhülse aufnimmt.

5. Vorrichtung nach einem der vorangehenden Ansprüche, dadurch gekennzeichnet, daß wenigstens eines die Septa (16, 18) an seinem nach außen weisenden Ende am Rand von einem angeschrägten Teil des Gehäusedeckels (10) oder der Wandung einer Septumhülse überragt wird.

6. Vorrichtung nach einem der vorangehenden Ansprüche, dadurch gekennzeichnet, daß wenigstens eine der Dichtungen (32, 34) an seinem zum Nadelstopp (36, 38) weisenden Ende mit einem plattenartigen Ansatz versehen ist, der über den Durchmesser der Dichtung (32, 34) an diesem Ende hinausragt.

7. Vorrichtung nach einem der vorangehenden Ansprüche, dadurch gekennzeichnet, daß das Septum zur Nachfüllung (16) eine relativ dünne Dichtung (32) auf dem Nadelstopp (38) besitzt und eine Nadel (20) mit einer seitlichen Öffnung in geringer Distanz zur Spitze der Nadel (20) zur Befüllung vorgesehen ist, und daß das Septum zur Bolusgabe (18) eine relativ dicke Dichtung (34) auf dem Nadelstopp (38) besitzt, und eine Nadel (22) mit einer seitlichen Öffnung in größerer Distanz zur Spitze der Nadel (22) zur Bolusgabe vorgesehen ist.

## Claims

1. Device for safe filling of at least two reservoirs under the skin surface, especially an infusion pump, comprising
- two septa (16, 18) of a soft material, so that two needles (20, 22) with lateral openings can be easily sticked in for filling,
- two needles (20, 22), at least one of them having an opening at a distance (A; B) from the point of the needle (20, 22),
- at least one space (42) each between further seals (32, 34) and the septa (16, 18), which is in communication with the reservoirs or catheters to be filled,
wherein these spaces (42) are spaced with different distances from a needle stop (36, 38),
characterized in that
- the septa (16, 18) are arranged adjacent to each other,
- the two needles have a lateral opening with a different distance (A, B) from the points, respectively, and
- at least one of the seals (16; 18; 32; 34) is shaped for blocking the opening of a needle, when the wrong needle (22; 20) is chosen for said septum.

2. Device according to claim 1, characterized in that the septa (16, 18) are made from a silicone rubber.

3. Device according to claim 1 or 2, characterized in that at least one of the septa (16, 18) is formed with a reduced diameter at its inwardly end and rests on a relatively thick support.

4. Device according to one of the preceding claims, characterized in that at least one of the septa (16, 18) is provided with a groove at its outwardly end, the groove taking up the casing lid (10) or the wall of the septum casing.

5. Device according to one of the preceding claims, characterized in that at least one of the septa (16, 18) is overtopped at the edge of its outwardly end by an inclined part of the casing lid (10) or the wall of the septum case.

6. Device according to one of the preceding claims, characterized in that at least one of the seals (32, 34) is provided with a plate-shaped nose at its end towards the needle stop (36, 38), which projects over the diameter of the seals (32, 34) at this end.

7. Device according to one of the preceding claims, characterized in that the septum (16) for refilling comprises a relatively thin seal (32) on the needle stop (38), and a needle (20) with a lateral opening at a small distance from the point of the needle (20) is provided for filling, and that the septum for a bolus dose (18) comprises a relatively thick seal (34) on the needle stop (38), and a needle (22) with a lateral opening at a larger distance from the point of the needle (22) is provided for the bolus dose.

## Revendications

1. Dispositif pour l'emplissage sûr d'au moins deux récipients sous la surface de peau, notamment d'une pompe d'infusion, avec
- deux septa (16, 18) d'une matière molle, de sorte que deux aiguilles (20, 22) avec des trous latéraux peuvent être introduites facilement pour l'emplissage,
- deux aiguilles (20, 22) dont au moins l'une a un trou à une distance (A; B) du bout de l'aiguille (20; 22),
- au moins chaque fois un espace (42) entre les autres joints (32, 34) et les septa (16, 18), qui est connecté aux récipients ou cathéters à remplir,
ces espaces (42) ayant de différentes distances d'un arrêt d'aiguille (36, 38),
caractérisé par le fait que
- les septa (16, 18) sont arrangés l'un à côté de l'autre,
- chacune des deux aiguilles a un trou latéral à distance différente (A; B) des bouts,
- et, au moins un des joints (16; 18; 32; 34) est formé de telle sorte qu'il bloque le trou dans une aiguille en cas où on choisit la fausse aiguille (22; 20) pour le septum.

2. Dispositif selon revendication 1, caractérisé par les septa (16, 18) étant faits de caoutchouc de silicones.

3. Dispositif selon revendication 1 ou 2, caractérisé par le fait qu'au moins un des septa (16, 18) a un diamètre réduit à son bout dirigé vers l'intérieur et est fixé sur un support relativement épais.

4. Dispositif selon une des revendications précédentes caractérisé par le fait qu'au moins un des septa (16, 18) est pourvu d'une cannelure au bout dirigé vers l'extérieur, la cannelure recevant le couvercle de la caisse (10) ou la paroi d'une douille du septum.

5. Dispositif selon une des revendications précédentes, caractérisé par le fait qu'au moins un des septa (16, 18) est dépassé au bord de son bout, qui est dirigé vers l'extérieur, par une partie inclinée du couvercle de la caisse (10) ou de la paroi d'une douille du septum.

6. Dispositif selon une des revendications précédentes, caractérisé par le fait qu'au moins un des joints (32, 34) est pourvu à son bout qui est dirigé vers l'arrêt d'aiguille (36, 38) d'une rallonge en forme de plaque, qui dépasse à ce bout le diamètre du joint (32, 34).

7. Dispositif selon une des revendications précédentes, caractérisé par le fait que le septum (16) pour le remplissage a un joint relativement mince sur l'arrêt d'aiguille (38) et une aiguille (20) avec un trou latéral à petite distance au bout de l'aiguille (20) est prévu pour le remplissage, et que le septum pour l'application d'un bolus (18) a un joint (34) relativement épais sur l'arrêt d'aiguille (38), et une aiguille (22) avec un trou latéral à distance plus grande au bout de l'aiguille (22) est prévue pour l'application d'un bolus.
